# EUROPEAN PATENT SPECIFICATION

(11) **EP 0 418 626 B1**
(45) Date of publication and mention of the grant of the patent: **29.12.1993**
(21) Application number: 90116878.1
(22) Date of filing: 03.09.1990
(51) Int. Cl.: C07K 13/00, A61K 39/108, A61K 39/295, A61K 39/245, C12N 15/62, C12N 15/31, C12N 15/38

(54) **Fused proteins and production thereof**
Fusionsproteine und Herstellung davon
Protéines fusionnées, et leur production

(30) Priority: 08.09.1989 JP 233728/89
(43) Date of publication of application: 27.03.1991
(73) Proprietor: Takeda Chemical Industries, Ltd., Chuo-ku, Osaka (JP)
(72) Inventor: Fujisawa, Yukio, Kobe, Hyogo 658 (JP); Hinuma, Shuji, Suita, Osaka 565 (JP); Mayumi, Aki, Osaka-sayama, Osaka 589 (JP); Yamamoto, Tatsuo, Kashiwa, Chiba 277 (JP)
(74) Representative: von Kreisler, Alek, Dipl.-Chem.

(56) References cited:
- EP-A- 0 372 928
- WO-A-86/06635
- WO-A-89/01041
- WO-A-89/02924
- INFECTION AND IMMUNITY, vol. 57, no.4, April 1989, p. 1347-1350, American Society for Microbiology, F. Schödel et al "Construction of a plasmid for expression of foreign epitopes as fusion proteins with subunit B of Escherichia coli heat-labile enterotoxin."
- ABSTRACTS OF THE ANNUAL MEETING OF THE AMERICAN SOCIETY FOR MICROBIOLOGY, 1988, p. 316, abstract no. S-6; A.M. Childress et al "Construction of a chimeric plasmid expressing glycoprotein B (gB), of herpes simplex virus type 1 and heat-labile enterotoxin (LT-B) of Escherichia coli."
- JOURNAL OF BACTERIOLOGY, vol. 169, no. 11, November 1987, p. 5201-5208, American Society for Microbiology; L.-M. Guzmann-Verduzco et al "Fusion of Escherichia coli heat-stable enterotoxin and heat-labile enterotoxin B subunit."

## Description

The present invention relates to techniques for producing fused proteins useful as immunogens for therapeutic and preventive vaccines and techniques for bioactive fused protein useful for treatment of various diseases by expressing genes for fused proteins of heat-labile enterotoxin B subunit (LTB) and a protein heterologous to heat-labile enterotoxin (LT), using recombinant techniques.

Surfactants have been used for enhancing absorption of foreign or bioactive substances (proteins) through nasal mucosa tissues, and choleratoxin has been studied for the purpose of immunization with foreign antigens through nasal mucosa tissues. However, it has been desired to develop a protein which enhances the absorption of foreign or bioactive sustances through nasal mucosa tissues and which is suitable for the immunization through nasal mucosa tissues.

With the object of preparing an antigen suitable for the absorption and immunization of a foreign or bioactive protein through nasal mucosa tissues by targetting the foreign or bioactive protein topically, the present inventors have conducted investigations. As a result, the present inventors have discovered that fused protein obtained by combining LTB with a heterologous protein by genetic engineering techniques can attain this object.

### SUMMARY OF THE INVENTION

Thus, the present invention relates to (1) a fused protein obtained by combining LTB with a protein heterologous to LT by genetic engineering techniques, (2) a recombinant DNA segment containing a nucleotide sequence coding for the fused protein described in (1), (3) a transformant harboring the recombinant DNA segment described in (2), (4) a method for producing the fused protein described in (1) which comprises cultivating the transformant described in (3), producing and accumulating the fused protein in a culture, and collecting the fused protein.

### BRIEF DESCRIPTION OF THE DRAWINGS

Fig. 1 is a representation showing an example of an amino acid sequence of a surface protein gD gene of HSV-1 strain Miyama;
Fig. 2 is a representation showing an example of a nucleotide sequence corresponding to the amino acid sequence shown in Fig. 1;
Fig. 3 is a representation showing an example of an amino acid sequence of a surface protein gB gene of the HSV-1 strain Miyama;
Fig. 4 is a representation showing an example of a nucleotide sequence corresponding to the amino acid sequence shown in Fig. 3;
Fig. 5 shows an example of a nucleotide sequence of a surface protein gB of HSV-1 strain KOS, and an amino acid sequence deduced therefrom;
Fig. 6 shows an example of a nucleotide sequence of a surface protein of HSV-1 strain F, and an amino acid sequence deduced therefrom;
Fig. 7 is a schematic representation showing the construction of plasmid pHSG396SgD;
Fig. 8 is a schematic representation showing the construction of a truncated gD gene of HSV-1;
Fig. 9 is a schematic representation showing the construction of an expression plasmid for animal cells of the truncated gD gene of HSV-1;
Fig. 10 is a schematic representation showing the construction of an expression plasmid of a fused protein gene according to the present invention;
Fig. 11 is a schematic representation showing the construction of plasmid pHDTneol.

### DESCRIPTION OF THE PREFERRED EMBODIMENTS

The fused protein comprises heat-labile enterotoxin B subunit (LTB) and a protein which is heterologous to heat-labile enterotoxin (heterologous protein).

The heterologous protein includes, for example, antigens (proteins or polypeptides) used for vaccines, and bioactive substances (proteins or polypeptides).

The antigens used for vaccines include antigens of viruses whose hosts are animals, such as antigens of herpesviruses including herpes simplex virus (HSV), varicella-zoster virus (VZV) and cytomegalovirus (CMV); antigens of retroviurses including human immunodeficiency virus (HIV) and adult T cell leukemia virus (HTLV-I); antigens of hepadonaviruses including hepatitis B virus (HBV); antigens of togaviruses including non-A, non-B hepatitis virus (HCV and HEV) and Japanese encephalitis virus; antigens of picornaviruses including hepatitis A virus (HAV); antigens of orthomyxoviruses including influenza virus; antigens of parvoviruses, antigens of papovaviruses; antigens of adenoviruses; antigens of poxviruses; antigens of reoviruses; antigens of paramyxoviruses; antigens of rhabdoviruses; antigens of arenaviruses; and antigens of coronaviruses; antigens of pathogenic protozoa such as a malarial antigen; and antigens of pathogenic protozoa such as a malarial antigen; and antigens of pathogenic bacteria such as a Bordetella pertussis antigen.

The bioactive substances include (e.g. an animal other than human, a plant or a microorganism) foreign or human-derived bioactive peptides and proteins such as hormones including insulin, human growth hormone, gonadotropin, inhibin, prolactin; enzymes including serratiopeptidase; cytokines including interferon (IFN), IFN-α, IFN-β, IFN-γ, interleukin (IL), IL-1, IL-2, IL-3, IL-4, IL-5, IL-6, IL-7, granular colony stimulating factor, granular macrophage colony stimulating factor, macrophage colony stimulating factor and erythropoietin; and growth factors including basic fibloblast growth factor and epidermal cell growth factor.

In some embodiments of the present invention, the protein heterologous to heat-labile enterotoxin may be fused with heat-labile enterotoxin through a linker. Linkers for use in the present invention can comprise one amino acid residue or a peptide residue comprising 2 to about 30 amino acid residues (preferably one amino acid residue or a peptide chain comprising 2 to about 10 amino acid residues) selected from G, A, V, L, I, S, T, C, M, E, D, K, R, H, F, Y, W, P, N and Q.

As an example of one of the many fused proteins disclosed herein, a fused protein of an HSV surface protein which is an HSV antigen with LTB will hereinafter be described. As the HSV surface protein, glycoproteins gD and gB lacking transmembrane domains are advantageously used.

Preferably, in accordance with the present invention, there are provided (1) a fused protein obtained by combining gD lacking transmembrane domain with LTB (I), or a fused protein obtained by combining gB lacking transmembrane domain with LTB (II); (2) a recombinant DNA containing a nucleotide sequence coding for the fused protein (I) or (II) (III or IV, respectively); (3) a transformant harboring the recombinant DNA (III) or (IV); (4) a method for producing the fused protein (I) or (II) which comprises cultivating the transformant harboring the recombinant DNA (III) or (IV), producing and accumulating the fused protein (I) or (II) in a culture, and collecting the fused protein.

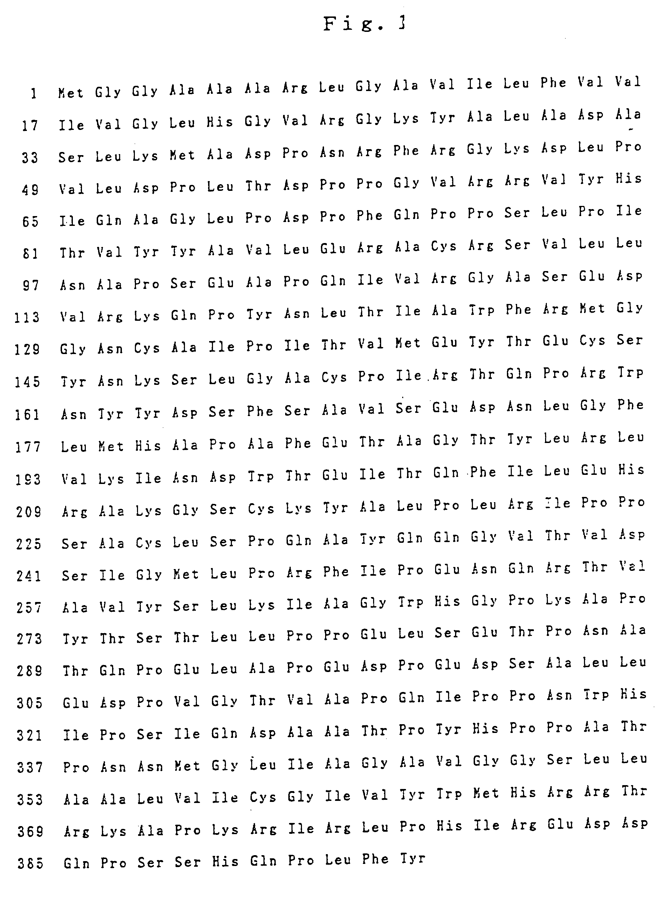

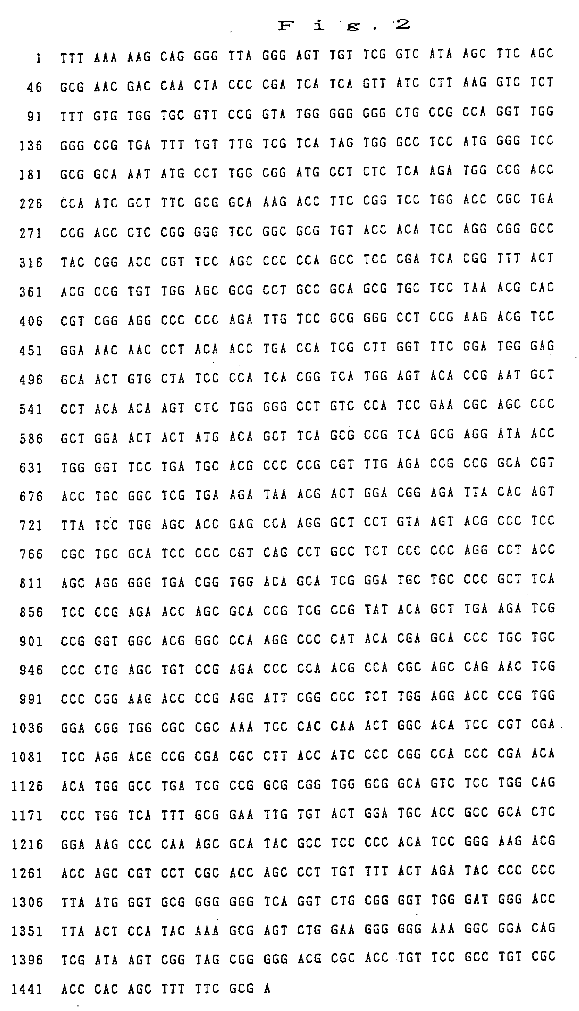

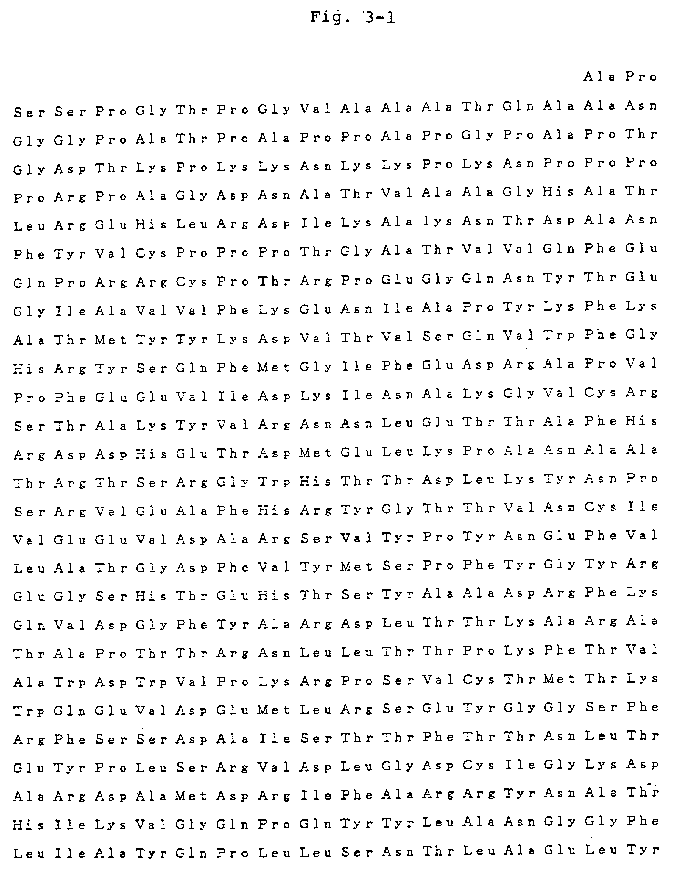

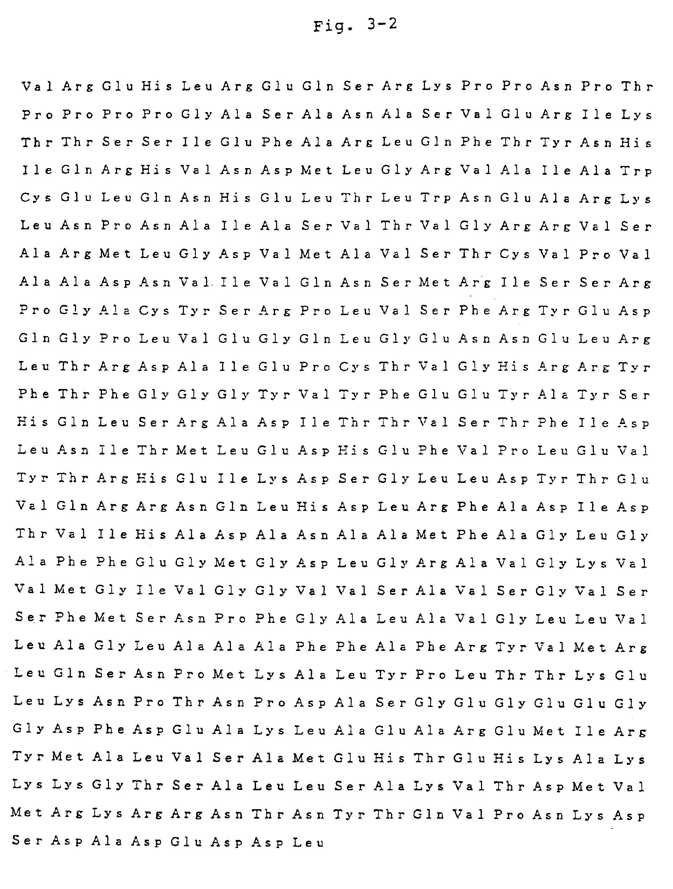

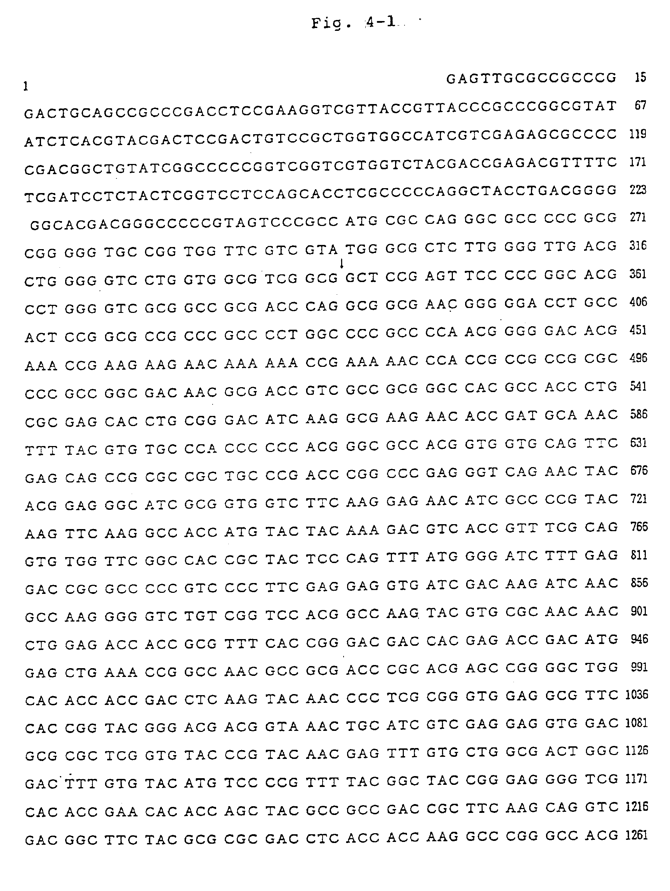

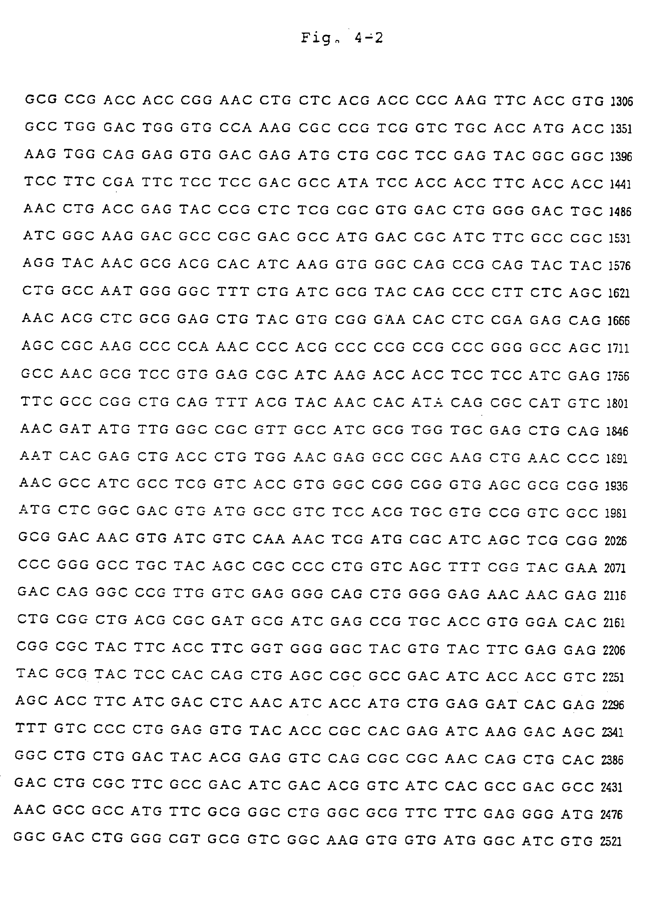

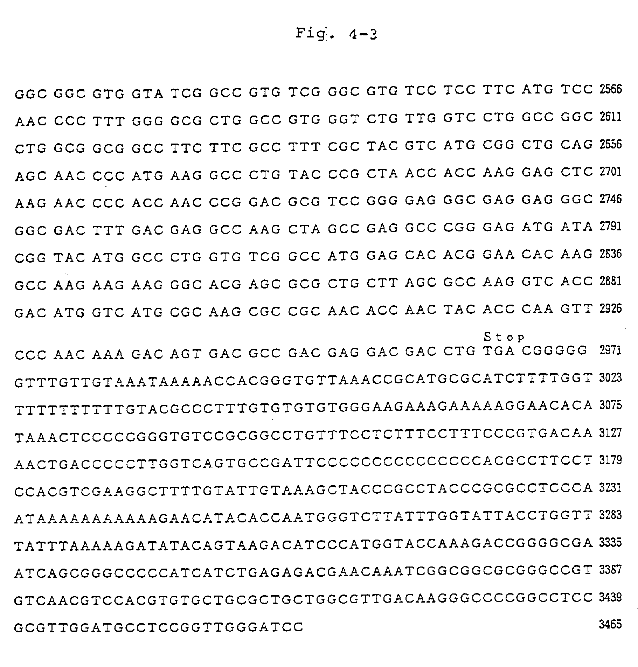

As surface protein genes of HSV, there can be used, for example, gD and gB genes of various HSV-1 strains such as HSV-1 strain Miyama. Antigenic fragments of the proteins expressed by such genes are also included. Examples of the gD include a polypeptide having the amino acid sequence shown in Fig. 1 (surface protein gD of HSV-1 strain Miyama, Japanese Patent Application No. 63-180114/1988). The essential portion of this amino acid sequence is from Lys of No. 26 to Ala of No. 302. Examples of the DNAs containing the nucleotide sequence coding for this gD gene include a DNA having a nucleotide sequence corresponding to the nucleotide sequence shown in Fig. 2. The portion from No. 186 to No. 1016 thereof corresponds to the essential portion. Examples of the gB include a polypeptide having the amino acid sequence shown in Fig. 3 (surface protein gB of HSV-1 strain Miyama, Japanese Patent Application No. 1-158238/1989 filed on June 22, 1989 and Japanese Patent Application No. 1-308941/1989 filed on November 30, 1989). The essential portion thereof is from Ala of No. 1 to Asp of No. 293. Examples of the DNAs containing the nucleotide sequence coding for this gB gene include a DNA having a nucleotide sequence corresponding to the nucleotide sequence shown in Fig. 4. The portion from No. 341 to No. 1219 thereof corresponds to the essential portion. As used herein corresponding to permits, additions, deletions and substitutions of nucleotides may be made so long as the protein produced by the gene will still by functional.

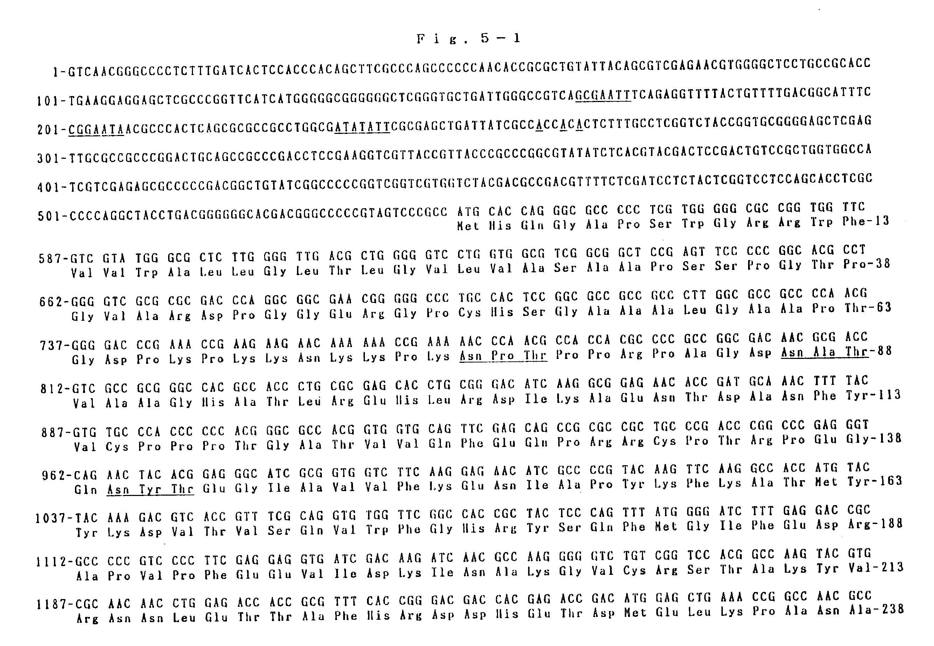

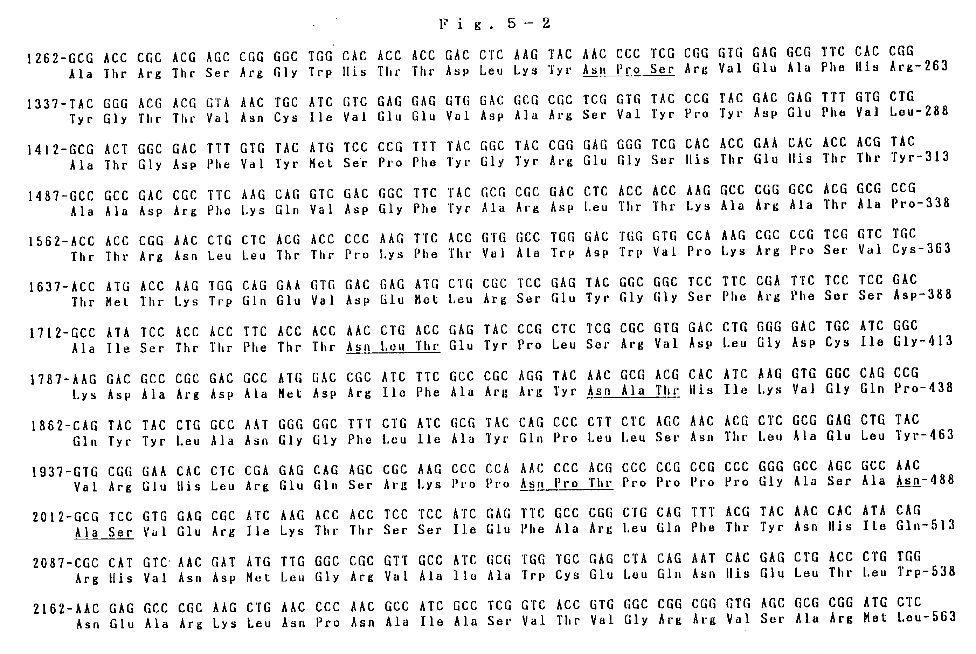

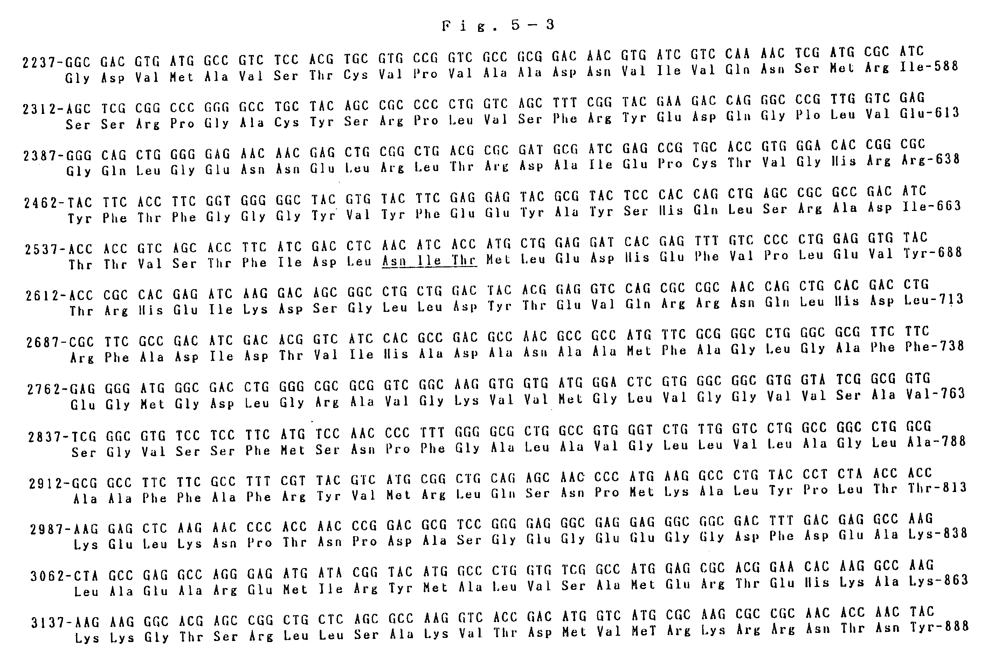

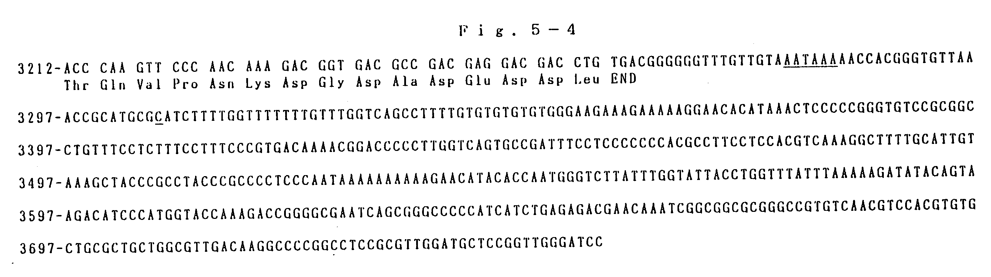

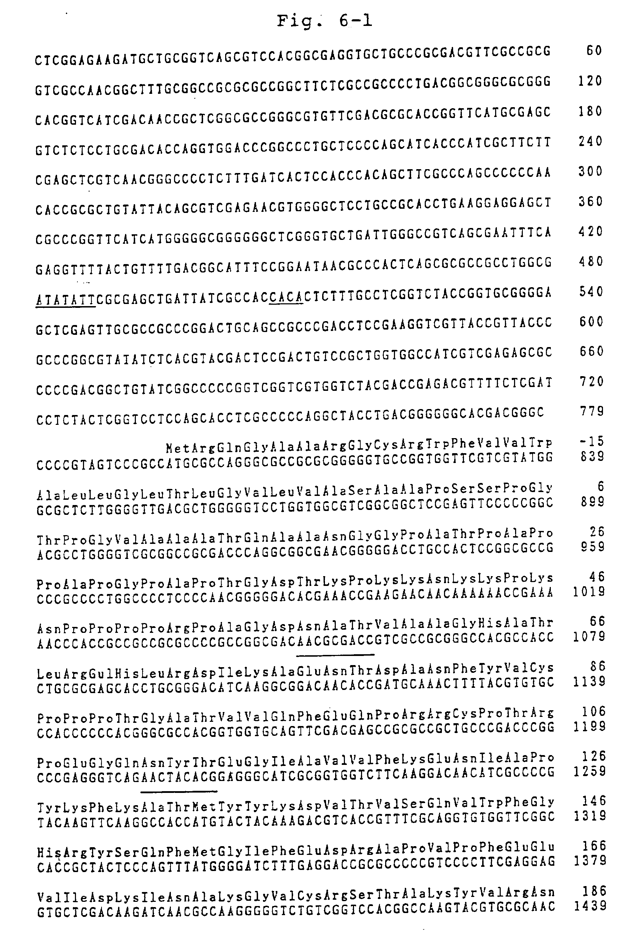

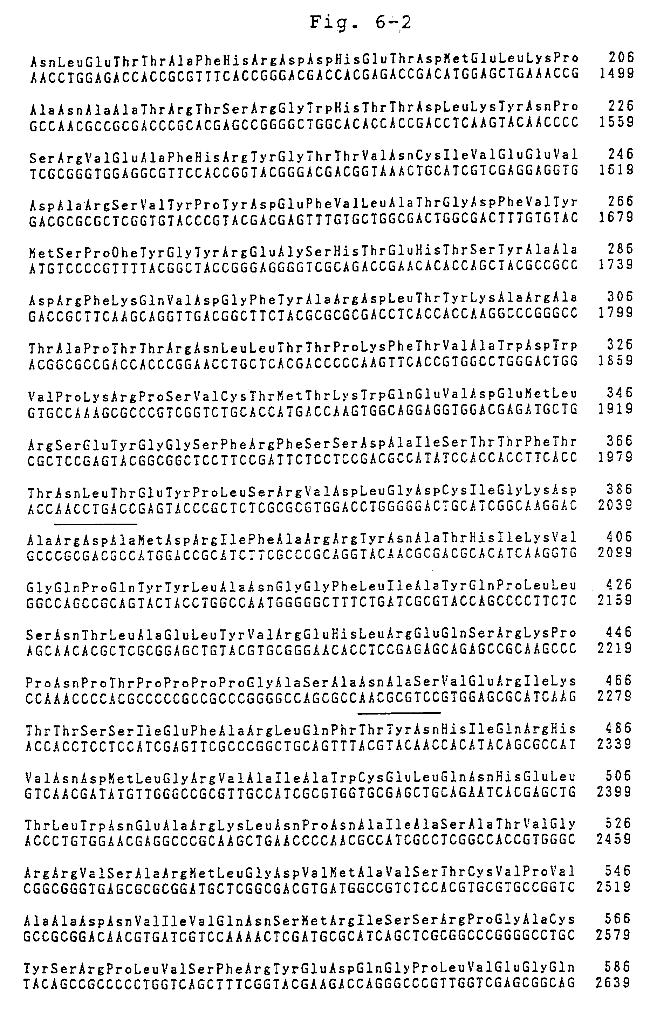

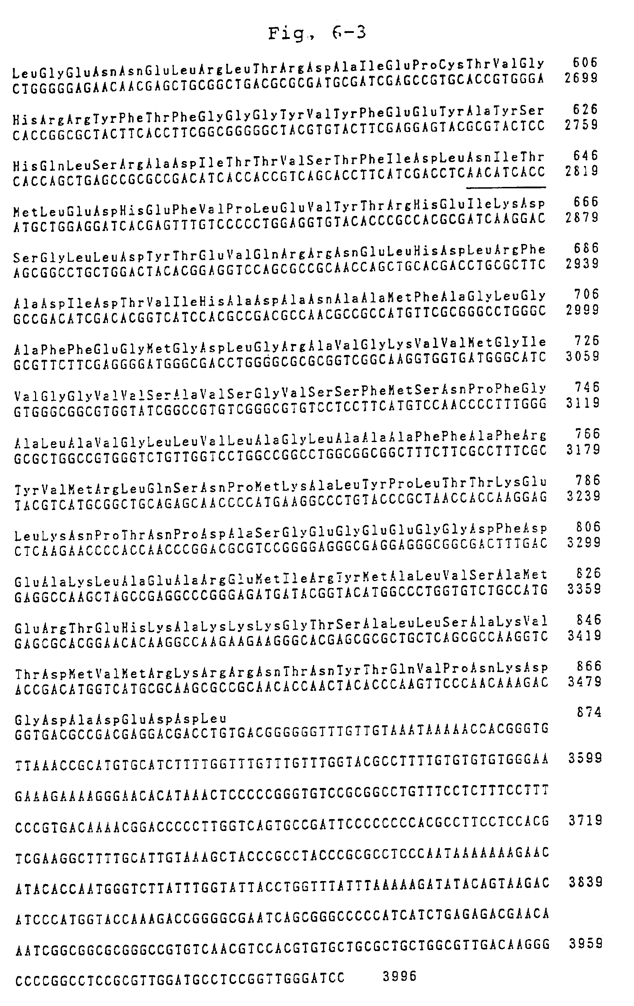

The gB genes further include, for example, genes having the nucleotide sequences and the amino acid sequences deduced therefrom shown in Fig. 5 [surface protein gB of HSV-1 strain KOS, D. J. Bzik et al., Virology, 133, 301 (1984)] and Fig. 6 [surface protein gB of HSV-1 strain F, P. E. Pellet et al., J. Virol., 53, 243 (1985)]. The LTB genes are combined with these genes, preferably with the truncated gD or gB gene lacking the coding regions of the transmembrane domains, whereby the fused protein genes can be constructed.

Any LTB gene can be used as long as it codes for an LTB active substance, and includes functional fragment thereof. The LTB active substance may be any LTB as long as it has LTB activity, namely the adjuvant activity. Examples of such substances include natural LTB produced in enterotoxigenic Escherichia coli separated from humans or pigs, recombinant LTB produced by recombinant technology and their related substances. In particular, B subunit (LThB) of LT produced in enterotoxigenic E. coli separated from humans (LTh) is preferable. When the LTB described above and the related substances thereof are proteins, they may have sugar chains or not.

As DNAs coding for LTB used for the present invention, there can be used any DNA segment coding for LTB produced by enterotoxigenic E. coli. The DNAs may be natural ones or synthetic ones, and may be a LTB gene or a portion thereof.

A DNA segment coding for LTB used for the present invention, for example, may be prepared from plasmid pJYL2299 containing LTh gene [T. Yamamoto and T. Yokota, J. Bacteriol., 143, 652 (1980)] using appropriate restriction enzymes. DNAs coding for LThB may be prepared in large amount by amplification of the subcloning plasmid in E. coli.

The recombinant DNA segment (expression plasmid) containing the nucleotide sequence coding for the fused protein (I) or (II) of the present invention can be prepared, for example, by the following processes.
(a) A desired truncated gene is cut out from a plasmid in which the gD or gB gene of HSV-1 strain Miyama has been cloned. It can also be chemically synthesized.
(b) An appropriate linker is added thereto as needed, followed by construction of a fused gene in which an LTB gene is linked to the 3'-terminal portion of the DNA.
(c) The resulting fused protein gene is ligated downstream from a promoter in an expression vector.

In the present invention, any vector (for example, plasmid) may be used as long as it can be replicated in an eucaryotic cell as a host. When the host is yeast, examples of such vectors include pSH19 [S. Harashima et al., Mol. Cell. Biol., 4. 771 (1984)] and PSH19-1 (European Patent Publication No. 0235430), and a vehicle for expression of foreign genes is obtained by inserting a promoter therein. When the host is an animal cell, the vehicle for expression of foreign genes is obtained, for example, by inserting an SV40-derived promoter, a retrovirus promoter or the like in pBR322.

As the promoter used in the present invention, any promoter is usable as long as the promoter is suitable for expression in the host used for the gene expression. When the host is yeast, it is preferred that a GLD (GAPDH) promoter, a PHO5 promoter, a PGK promoter, an ADH promoter, a PHO81 promoter and the like are used. When the host is an animal cell, an SV40-derived promoter, a retrovirus promoter and the like are preferably used.

The promoters can be prepared enzymatically from the corresponding genes. They can also be chemically synthesized.

By using the vector containing the recombinant DNA thus constructed, the eucaryotic cell is transformed.

The host includes, for example, yeast and animal cells.

Examples of the yeast include Saccharomyces cerevisiae AH22R⁻, NA87-11A and DKD-5D and Schizosaccharomyces pombe ATCC38399 (h⁻ leul-32) and THl68(h⁹⁰ ade6-M210 ural leul) [M. Kishida and C. Shimada, Current Genetics, 10, 443(1986)].

Examples of the animal cells include adherent cells such as monkey COS-7 cell, monkey Vero cell, Chinese hamster ovary (CHO) cell, mouse L cell and human FL cell, and non-adherent cells such as mouse myelomacell (such as SP2/0), mouse YAC-1 cell, mouse methA cell, mouse P388 cell and mouse EL-4 cell.

The transformation of the yeast is carried out according to, for example, the method described in Proc. Natl. Acad. Sci. U.S.A., 75, 1929 (1978). The transformation of the animal cell is carried out according to, for example, the method described in Virology, 52, 456 (1973).

The transformants (recombinants) thus obtained are cultivated by per se known methods.

When the transformants in which the host is yeast are cultivated, there is used, for example, Burkholder minimum medium [K. L. Bostian et al., Proc. Natl. Acad. Sci. U.S.A., 77, 4505 (1980)] as a medium. The pH of the medium is preferably adjusted to about 5 to 8. The cultivation is usually carried out at about 20 to 35+C for about 24 to 72 hours, with aeration or agitation if necessary.

When the transformants in which the host is an animal cell are cultivated, there can be used as the medium, for example, about 5 to 20% fetal calf serum-containing MEM medium [Science, 122, 501 (1952)], DMEM medium [Virology, 8, 396 (1959)], RPMI1640 medium [The Journal of the American Medical Association, 199, 519 (1967)] and 199 medium [Proceeding of the Society for the Biological Medicine, 73, 1 (1950)]. The pH is preferably about 6 to 8. The cultivation is usually carried out at about 30 to 40°C for about 15 to 60 hours, with aeration or agitation if necessary.

In the present invention, the fused proteins having both the HSV surface antigenicity and the LTB activity can be separated and purified by appropriate combinations of per se known separating and purifying methods. These known separating and purifying methods include methods utilizing a solubility such as salt precipitation and solvent precipitation, methods mainly utilizing a difference in molecular weight such as dialysis, ultrafitration, gel filtration and SDS-polyacrylamide gel electrophoresis, methods utilizing a difference in electric charge such as ion-exchange column chromatography, methods utilizing specific affinity such as affinity chromatography, methods utilizing a difference in hydrophobicity such as reverse-phase high performance liquid chromatography and methods utilizing a difference in isoelectric point such as isoelectro focusing electrophoresis.

For the separation of fused proteins having both the HSV surface antigenicity and the LTB activity, a combination of ion-exchange chromatography and gel permeation chromatography. As fillers for ion exchange chromatography, cationic exchange resins using a sulfo-group as an exchanging group such as ST-Toyopearl 650 M (Toyo Soda, Japan) are preferable. Sovents for equilibriation of columns include 20 mM phosphate buffer (pH 5.8), and eluate solvents include 20 mM phosphate buffer containing 0 to 1 M NaCl (pH 5.8). As fillers for gel permeation chromatography, porous particles such as Sephacryl 5-300HR (TM) which may isolate a protein of about 1X10⁴ to 5X10⁶ molecular weight, are preferable. As solvents for gel permeation chromatography, 5 to 20 mM phosphate buffer containing 0.5 to 1.5 % NaCl (pH 6.8 to 7.2) is preferable. The fused protein can be purified as described above so that it is substantially free of contaminants. This fused protein can then be administered therapeutically.

The fused protein of a heterologous protein other than HSV surface antigen protein and LTB can be prepared using a gene (DNA) coding for that heterologous protein in lieu of the HSV surface antigen protein gene, according to the methods described above.

The antigen protein-LTB fused proteins and bioactive protein-LTB fused proteins are remarkably efficient for targetting to nasal mucosa tissues as compared with unfused proteins. LTB has not only binding activity specific to glycolipids on mucosal epidermal cell membranes but also activity to enhance the abosorption of a coexistent protein through mucosal tissues. The activity increases in case that LTB is fused with an objective protein. As a result, the antigen protein-LTB fused protein can induce immune response efficiently through nasal mucosa tissues, and the bioactive protein-LTB can enhance the absorption of the bioactive protein through nasal mucosa tissues.

The antigen protein-LTB fused protein can be administered pernasally as well as intramuscularly, subcutaneously or intracutaneously, as vaccines for prevention or treatment of infection of viruses, pathogenic protozoas or pathogenic bacteria. The bioactive protein-LTB fused protein can be administered pernasally. The per vaginam administration of an HSV antigen-LTB fused protein is also efficient. One would administer a therapeutically effective amount of the fused protein. This can be determined empirically. Typical dosages would range from 1 µg to 1 mg per body.

When bases, amino acids and so on are indicated by the abbreviations in this specification and the drawings, the abbreviations adopted by IUPAC-IUB Commission on Biochemical Nomenclature or commonly used in the art are employed. For example, the following abbreviations are used. When the optical isomer is capable of existing with respect to the amino acids, the L-form is represented unless otherwise specified.
- DNA: : Deoxyribonucleic acid
- A: : Adenine
- T: : Thymine
- G: : Guanine
- C: : Cytosine
- SDS: : Sodium dodecyl sulfate
- Gly: : Glycine (G)
- Ala: : Alanine (A)
- Val: : Valine (V)
- Leu: : Leucine (L)
- Ile: : Isoleucine (I)
- Ser: : Serine (S)
- Thr: : Threonine (T)
- Cys: : Cysteine (C)
- 1/2 Cys: : Half cysteine
- Met: : Methionine (M)
- Glu: : Glutamic acid (E)
- Asp: : Aspartic acid (D)
- Lys: : Lysine (K)
- Arg: : Arginine (R)
- His: : Histidine (H)
- Phe: : Phenylalanine (F)
- Tyr: : Tyrosine (Y)
- Trp: : Tryptophan (W)
- Pro: : Proline (P)
- Asn: : Asparagine (N)
- Gln: : Glutamine (Q)
- Ap^{r}: : Ampicillin-resistant gene
- Tc^{r}: : Tetracycline-resistant gene

With respect to the proteins of the present invention, a portion of the amino acid sequence may be modified, namely there may be addition, elimination or substitution by a different amino acid(s). Furthermore, functional fragments of the proteins may also be used.

The present invention will hereinafter be described in detail with the following Reference Examples and Examples. It is understood of course that these Reference Examples and Examples are merely illustrative and are not intended to limit the scope of the invention.

Transformant CHO-HDT-2-35 obtained in Example 6 described below and harboring plasmid pHDTdhfr2 was deposited with the Fermentation Research Institute, Agency of Industrial Science and Technology, Ministry of International Trade and Industry, Japan (FRI) under the accession number FERM BP-2590 on September 8, 1989. This microorganism was also deposited with the Institute for Fermentation, Osaka, Japan (IFO) under the accession number IFO 50209 on August 24, 1989.

Trasformant Sp-neo-HDT-13-71 obtained in Example 8 described below and harboring plasmid pHDTneol was deposited with the FRI under the accession number FERM BP-3071 on August 17, 1990. This microorganism was also deposited with the Institute for Fermentation, Osaka, Japan (IFO) under the accession number IFO 50254 on August 8, 1990.

Transformant Escherichia coli DH1/pHSD BJ-1 harboring plasmid pHSD BJ-1 described in Reference Example mentioned below was deposited with the FRI under the accession number FERM BP-1784 on March 9, 1988. This microorganism was also deposited with the IFO under the accession number IFO 14730 on February 23, 1988.

### Reference Example 1

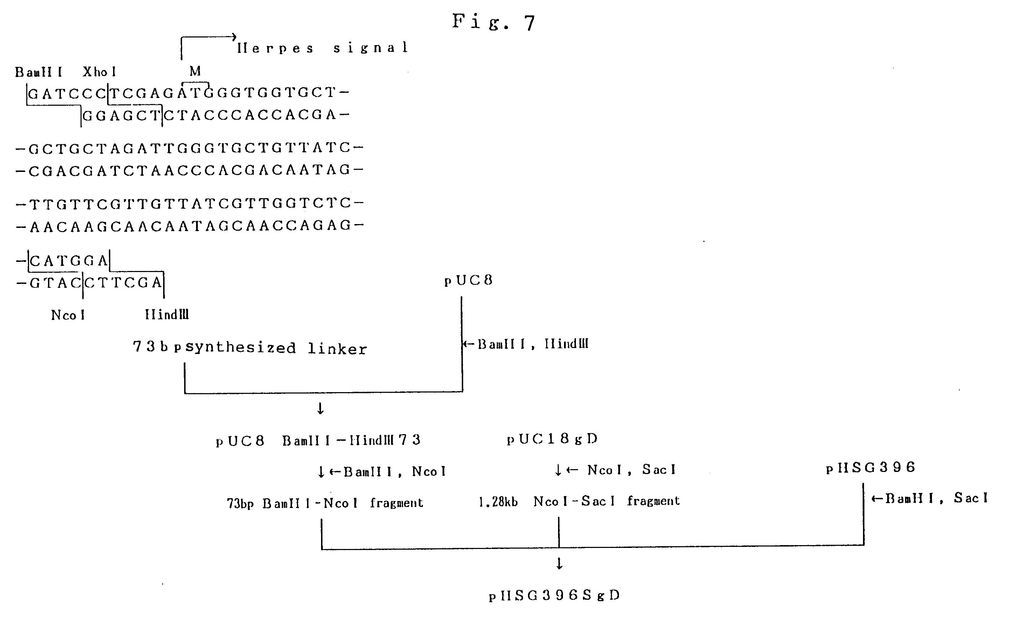

### Preparation of Plasmid pHSG396SgD (Fig. 7)

A DNA coding for the 20 amino acid residues from the N-terminus of gD, namely the 73-bp DNA fragment shown in Fig. 7 was chemically synthesized, and inserted into vector pUC8 digested with BamHI and HindIII.

The resulting pUC8 BamHI-HindIII73 was digested with BamHI and NcoI to obtain a 73-bp fragment. On the other hand, a NcoI-SacI DNA fragment of about 1.28 kb was obtained from cloning plasmid pUC18gD having an HindIII-NruI fragment [plasmid pHSD BJ-1 (IFO 14730, FERM BP-1784 origin] of about 1.4 kb containing the gD-coding region of HSV. The above 73-bp fragment and the above NcoI-SacI DNA fragment were reacted with a BamHI-SacI digest of plasmid vector pHSG396 (Takara Shuzo) to prepare subcloning plasmid pHSG396SgD.

### Reference Example 2

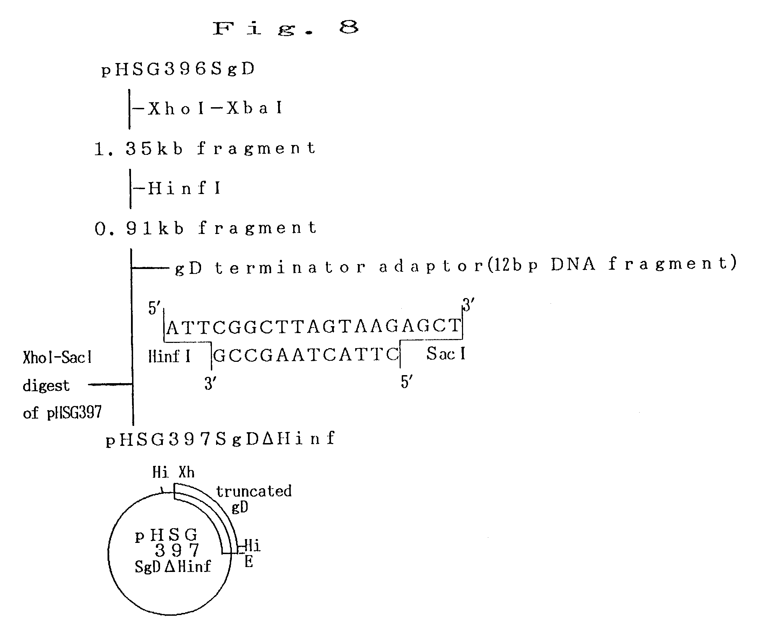

### Construction of HSV-1 Truncated gD Gene (Fig. 8)

The plasmid vector pHSG396SgD (Reference Example 1) having the HSV-1 strain Miyama gD gene was digested with restriction enzymes XhoI and XbaI to obtain a DNA fragment of about 1.35 kb, followed by further digestion with restriction enzyme HinfI to obtain an XhoI-HinfI fragment of about 0.91 kb. A 12-bp DNA fragment shown in Fig. 8 containing a stop codon was chemically synthesized, and reacted with the above XhoI-HinfI fragment and an XhoI-SacI digest of plasmid vector pHSG397 (Takara Shuzo) to prepare subcloning plasmid pHSG397SgDΔHinf.

### Example 1

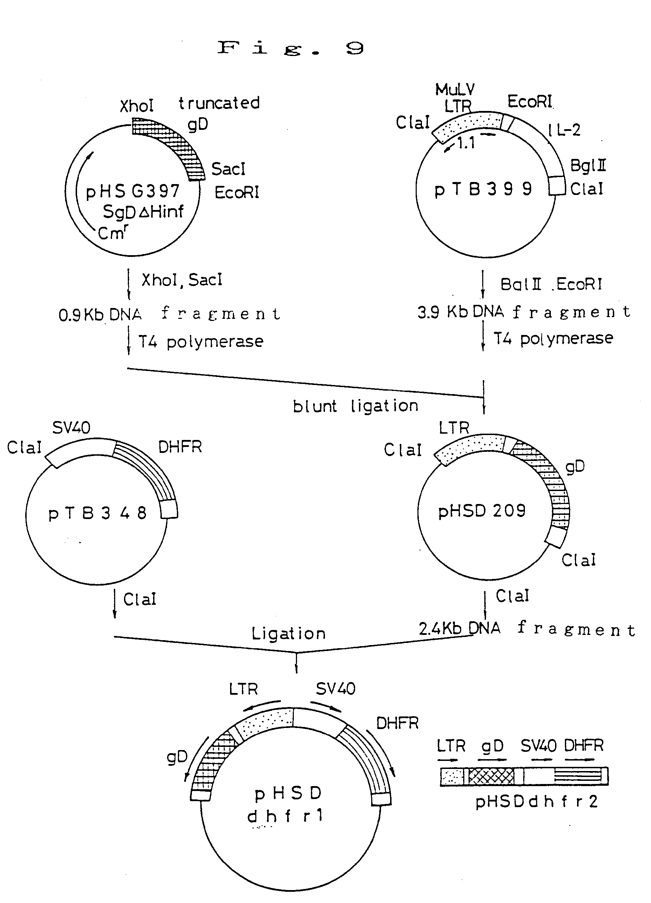

### Construction of Gene Expression Plasmid in Animal Cells for HSV-1 Trucated gD Gene (Fig. 9)

The plasmid pHSG397SgDΔHinf shown in Reference Example 2 was digested with restriction enzymes XhoI and SacI, and then T4 DNA polymerase was allowed to react on the digest to obtain a fragment of about 0.9 kb containing the truncated gD gene, both ends of the fragment being flush.

Then, plasmid pTB399 [Japanese Patent Unexamined Publication No. 61-63282/1986; R. Sasada et al., Cell Structure and Function, 12, 205 (1987)] was digested with restriction enzymes EcoRI and BglII, and then T4 DNA polymerase was allowed to react on the digest to obtain a fragment of about 3.9 kb both ends of which are flush. The resulting fragment was reacted with the above fragment containing truncated gD in the presence of T4 DNA ligase to obtain expression plasmid pHSD209.

Then, in order to express the gene stably in CHO cells and to enable gene amplification, a fragment of about 2.4 kb which was obtained by digesting the plasmid pHSD209 with restriction enzyme ClaI was inserted into the ClaI site of plasmid pTB348 (refer to Japanese Patent Unexamined Publication No. 61-63282/1986) to obtain plasmids pHSDdhfr1 and pHSDdhfr2.

### Example 2

### Gene Expression of Truncated gD in Animal Cell

Using the plasmid pHSDdhfr1 constructed in Example 1, CHO cell DHFR⁻ strain [G. Urlaub and L. A. Chasim, Proc. Natl. Acad. Sci. U.S.A., 77, 4216-4220 (1980)] was transformed by the calcium phosphate method [C. M. Gorman et al, Science, 221, 551-553 (1983)] to obtain a transformant which was converted to DHFR⁺.

The resulting transformant CHO-HSD-1-7 was cultivated in Dulbecco MEM medium (Gibco) containing 10% fetal calf serum (Whittaker M. A. Bioproducts) so as to become confluent. Then, the medium was exchanged for a methionine-free medium, and 25 µCi/ml of ³⁵S-methionine was added thereto, followed by cultivation overnight.

After a supernatant of the culture was recovered, 5 µl/ml of supernatant of rabbit anti-HSV-1 (Maclntyre) serum (Dakopatt) was added and the mixture was incubated at 4°C for 2 hours. Protein A-Sepharose (Pharmacia) was added to the mixture, followed by incubation at 4°C for 2 hours, and by centrifugation to recover a precipitate. The precipitate was washed with a buffer containing 0.05% NP-40, and Laemmli buffer was added thereto, followed by heating at 100°C for 5 minutes. After cooling, a supernatant was recovered by centrifugation and subjected to SDS-polyacrylamide gel electrophoresis. After electrophoresis, the gel was dried, and subjected to autoradiography. As a result, it was revealed that products of about 43 to 30 k daltons which were reactive to anti-HSV-1 was produced.

### Example 3

### Purification of HSV-1 Truncated gD (t-gD) Expressed in Animal Cell

1.8 liter of the culture solution of CHO-HSD-1-7 strain cultivated by the method described in Example 2 was dialyzed against 36.0 liter of buffer (pH 8.0) containing 20 mM Tris at 4°C, for 16 hours. 336 g of ammonium sulfate was added gradually to 2 liter of the dialyzed solution and the mixture was stirred at 4°C for 2 hours.

The resulting solution was applied onto a Butyl-Toyopearl column (Toyo Soda Co. Ltd. Japan) equilibrated with 168 g/l ammonium sulfate-20 mM Tris bufer (pH 8.0), and then the column was washed with 1.0 liter of the same buffer. Subsequently, t-gD was eluted with a linear gradient (600 ml) from 168 g/l of ammonium sulfate - 20 mM Tris buffer (pH 8.0) to 20 mM Tris buffer (pH 8.0). About 140 ml of the eluate containing t-gD was concentrated to 14 ml using Centriprep 10 (Amicon, U.S.A.).

The truncated gD fraction purified with the above Butyl-Toyopearl column was dialyzed against distilled water, followed by filtration for sterilization to obtain 14 ml of a purified truncated gD sample (536 µg/ml).

### Example 4

### Characterization of HSV-1 Truncated gD Purified from Animal Cell

The following property of the purified truncated gD sample obtained in Example 3 was assayed.

The sample was subjected to SDS-polyacrylamide slab electrophoresis according to Laemmli's method [Nature, 227, 680 (1970)], and then to silver staining. As a result, the truncated gD protein was composed of molecules from 43,000 daltons to 30,000 daltons.

### Example 5

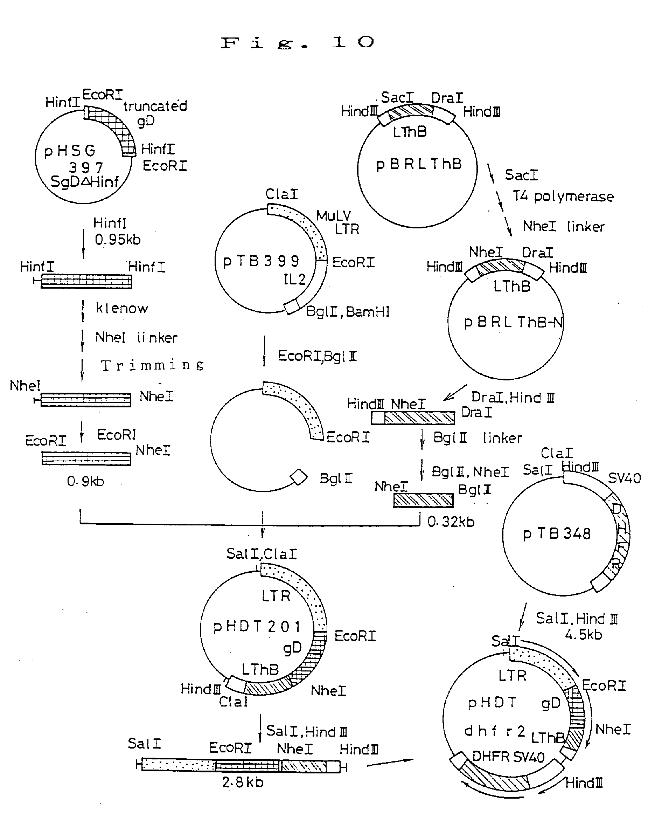

### Construction of Gene Expression Plasmid for Fused Protein Composed of HSV-1 truncated gD and LThB (Fig. 10)

pHSG397SgDΔHinf described in Reference Example 2 was digested with HinfI to obtain a DNA fragment of about 0.95 kb. The termini of the fragment were changed flush with a Klenow fragment, and NheI linkers were linked thereto, followed by digestion with NheI. The resulting fragment was further digested with EcoRI to isolate an EcoRI-NheI fragment of about 0.9 kb coding for truncated gD.

Plasmid pJYL2299 containing E. coli heat-labile toxin gene [T. Yamamoto et al., J. Bacteriol., 148, 983 (1981)] was digested with restriction enzyme HindIII to obtain a fragment of 0.77 kb containing B subunit. The resulting fragment was reacted with a digest of pBR322 with HindIII to obtain subcloning plasmid pBRLThB. The obtained plasmid was digested with restriction enzyme SacI, followed by reaction with T4 DNA polymerase and addition of NheI linker (pCGCTAGCG) (Pharmacia) using T4 DNA ligase to obtain plasmid pBRLThB-N. The plasmid was digested with restriction enzymes DraI and HindIII to obtain a DNA fragment of about 0.59 kb. ,To the fragment was added BglII linker (pGAAGATCTTC) (NEB) using T4 DNA ligase, followed by digestion with NheI and BgIII to obtain a NheI-BglII fragment of 0.32 kb containing LThB-coding region.

The above described 0.9 kb- and 0.32 kb- DNA fragments and an about 3.9 kb fragment obtained by EcoRI-BglII digestion of pTB399 [Japanese Patent Unexamined Publication No. 61-63282/1986; R. Sasada et al., Cell Structure and Function, 12, 205 (1987)] were reacted with each other to obtain expression plasmid pHDT201.

Then, in order to express the fused gene in CHO cells and to enable gene amplification, a fragment of about 2.8 kb comprising a gD-LThB fused protein gene which was obtained by digesting pHDT201 with SalI and HindIII was reacted with a fragment of about 4.5 kb which was obtained by digesting dihydrofolate reductase (DHFR) gene expression plasmid pTB348 (Refer to Japanese Patent Unexamined Publication 63282/1986) with SalI and HindIII to obtain plasmid pHDTdhfr2 (Fig. 10).

### Example 6

### Expression of Fused Protein Composed of HSV-1 truncated gD and LThB in Animal Cell

The plasmid pHDTdhfr2 constructed in Example 5 was introduced into CHO cell DHFR⁻ strain by the calcium phosphate method similar to Example 2, to obtain a transformant which was converted to DHFR⁺.

The resulting transformant CHO-HDT-2-35 (IFO 50209, FERM BP-2590) was cultivated by a similar method to Example 2, and then was isotope-labeled with ³⁵S-methionine, followed by immunoprecipitation of the gene product using a rabbit anti HSV-1 antibody and a goat anti choleratoxin antibody (LIST BIOLOGICAL LABORATORIES).

As a result, it was revealed that products of about 45 to 60 kdaltons which were reactive to both anti-HSV-1 and anti-choleratoxin antibodies were produced.

### Example 7

### Construction of Gene Expression Plasmid for Fused Protein Composed of HSV-1 Truncated gD and LThB in Myeloma Cell

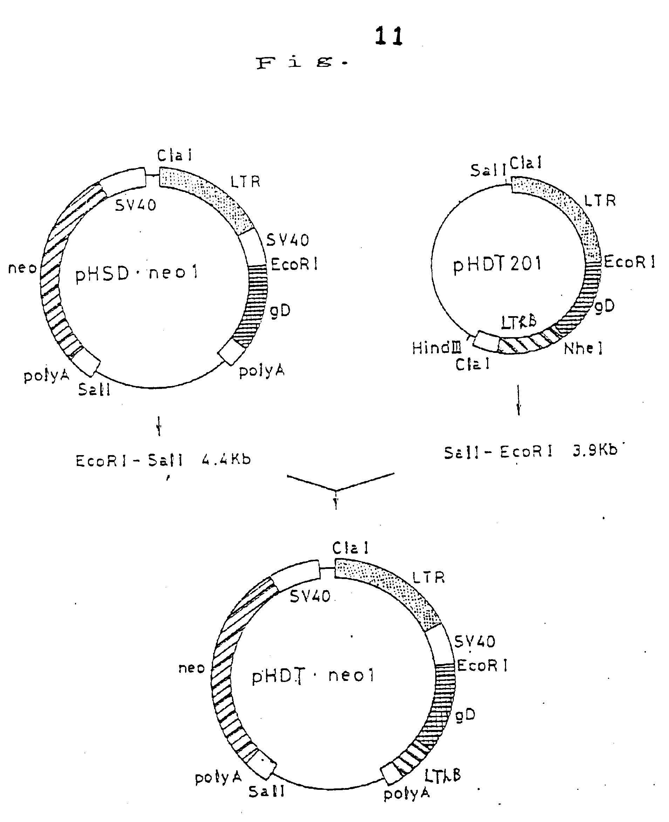

The plasmid pHDT201 constructed in Example 5 was digested with restriction enzyme SalI and EcoRI to obtain a fragment of about 3.9 kb containing a fused gene composed of truncated gD and LThB. On the other hand, the truncated gD expression plasmid pHSDneol (Japanese Patent Application No. 2-177258/1990) having the neomycin-resistant gene was digested with SalI and EcoRI to obtain a fragment of about 4.4 kb containing the neomycin-resistant gene. These two fragments were reacted with each other to obtain expression plasmid pHDTneol of the truncated gD-LThB fused gene having the neomycin-resistant gene (refer to Fig. 11).

### Example 8

### Gene Expression of Fused Protein Composed of HSV-1 Truncated gD and LThB in Myeloma Cell

Using the plasmid pHDTneol constructed in Example 7, mouse myeloma cell Sp2/0-Ag14 (Dainippon pharmaceutical) was transformed by electroporation using a Gene Pulser (Bio-Rad), followed by cultivation in RPMI1640 medium (Gibco) containing 200 µg/ml of G418 (Gibco) and 10% fetal calf serum to obtain G418-resistant transformants. A culture supernatant of the transformants was screened according to an enzyme immunoassay by a sandwich method using a microplate (Nunc) coated with rabbit anti-HSV-1 serum (Dakopatt) and biotinyl ani-HSV-1 & -2 antibody (Chemicon) to obtain clones in which truncated gD was expressed.

Of the clones, Sp-neo-HDT-13-71 relatively high in expression amount was cultivated in serum-free medium ASF104 (Ajinomoto), and 1 ml of a supernatant thereof was concentrated by Molcut (Millipore). Then, Laemmli buffer was added thereto to 50 µl, followed by heating at 100°C for 5 minutes. After cooling, SDS-polyacrylamide gel electrophoresis was conducted, and further the western blotting was carried out using rabbit anti-HSV-1 serum (Dakopatt) and goatanti-cholera toxin serum. As a result, a band recognized by both of the antibodies was specifically detected.

### Example 9

### Purification of Fused Protein Composed of Truncated gD and LThB (t-gD-LThB)

Transformant Sp-neo-HDT-13-71 obtairted in Example 8 was suspended in serum-free medium ASF1O4 (Ajinomoto) and cultivated at 37°C for 4 to 7 days in the presence of 5% CO₂. The culture was centrifuged at 4,000 rpm for 20 minutes with Hitachi CR26H centrifuge (Type 30 rotor) to give a culture supernatant (5.5 l). The supernatant was dialyzed against 20 mM phosphate buffer (pH 5.8) and filtered with No. 2 filter paper (Advantech Toyo). The filtrate was poured into a column (bed volume: 540 ml; ø4.1 x 41 cm) charged with Sp-Toyopearl 650M (Toso) equilibrated with 20 mM phosphate buffer (pH 5.8). The column was washed with 20 mM phosphate buffer (pH 5.8), and then t-gD-LThB was eluted with a gradient of 0 to 1 M NaCl (total 4 l). The t-gD-LThB fractions eluted by about 0.2 to 0.35 M NaCl were concentrated to a volume of 8.3 ml by ultrafiltration (Amicon, YM10), and the concentrate was subjected to a column (bed volume: 198 ml; ø1.6 x 98.5 cm) charged with Sephacryl S-300HR (Pharmacia) equilibrated with phosphate-buffered saline (PBS). The t-gD-LThB fractions were concentrated by ultrafiltration (Amicon, YM10) to obtain a purified sample (1.7,25 mg/0.845 ml).

### Example 11

### Immunogenicity of Fused Protein Composed of HSV-1 Truncated gD and LThB (t-gD-LThB)

t-gD-LThB obtained in Example 10 was administered in an amount of 16 µl/mouse to the internal nares of BALB/c mice (female, 8 weeks old, Charles River). After 5 weeks, blood was collected and serum sample was prepared. The internal nares were washed with PBS several times to give a washing. The anti-HSV antibodies in these samples were determined by the following method.

An inactivated HSV-coated microplate of a human anti-HSV antibody determination kit (Herpes Stat, Whittaker Bioproducts, Lot No. 002706) was blocked with PBS containing 20% FCS at room temperature for 2 hours, followed by washing 3 times with PBS containing 0.05% Tween 20 (PBS-Tween). To this plate was added 100 µl/well of the serum sample diluted with 20% FCS/40 mM Tris-HCl (pH 7.5)/5% NaCl/0.05% Tween 20, followed by incubation at room temperature for 1 hour. The plate was washed 6 times with PBS-Tween, and then 100 µl of a 100- to 1,000-fold dilution of a peroxidase-labeled anti-mouse IgG antibody or anti-mouse IgA antibody (Zymed Laboratories) was added to each well, followed by incubation at room temperature for 30 minutes. The plate was washed 6 times with PBS-Tween, and then 100 µl of a substrate solution [2 mg/ml o-phenylenediamine/0.02% H₂O₂/0.1 M citrate buffer (pH 4.5)] was added to each well, followed by reaction for 10 minutes. After 200 µl of 2 N sulfuric acid was added to each well to terminate color development, absorbance was measured at 492 nm.

As a result, it was proved that IgG and IgA antibodies against HSV could effectively be induced in blood and/or internal nares by the administration of t-gD-LThB to internal nares.

## Claims (Claims for the following Contracting State(s): AT, BE, CH, DE, DK, FR, GB, GR, IT, LI, LU, NL, SE)

1. A fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side.

2. A fused protein in accordance with claim 1, wherein the heat-labile enterotoxin B subunit is fused with the herpes simplex virus surface antigen through a linker.

3. A fused protein in accordance with claim 1, which is a fused protein comprising herpes simplex virus surface antigen and heat-labile enterotoxin B subunit.

4. A fused protein in accordance with claim 1, wherein the heat-labile enterotoxin B subunit is a product of enterotoxigenic Escherichia coli separated from a human.

5. A fused protein in accordance with claim 1, wherein the herpes simplex virus surface antigen is gD or gB of herpes simplex virus type I or type II.

6. A fused protein in accordance with claim 5, wherein the gD or gB is gD or gB lacking a transmembrane domain.

7. A fused protein in accordance with claim 1, which is a fused protein comprising gD lacking a transmembrane domain of herpes simplex virus type I and heat-labile enterotoxin B subunit.

8. A recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side.

9. A recombinant DNA segment in accordance with claim 8, wherein the fused protein is a fused protein comprising gD lacking a transmembrane domain of herpes simplex virus type I and heat-labile enterotoxin B subunit.

10. A transformant harboring a recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side.

11. A transformant in accordance with claim 10, wherein the fused protein is a fused protein comprising gD lacking a transmembrane domain of herpes simplex virus type I and heat-labile enterotoxin B subunit.

12. A transformant in accordance with claim 10, which has the characteristics of CHO-HDT-2-35 (FERM BP-2590).

13. A transformant in accordance with claim 10, which has the characteristics of Sp-neo-HDT-13-71 (FERM BP-3071).

14. A method for producing a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising cultivating a transformant harboring a recombinant DNA segment comprising a nucleotide sequence coding for the fused protein, producing and accumulating the fused protein in a culture, and collecting the fused protein.

15. A method in accordance with claim 14, wherein the fused protein is a fused protein comprising gD lacking a transmembrane domain of herpes simplex virus type I and heat-labile enterotoxin B subunit.

16. A method for purifying a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising cultivating a transformant harboring a recombinant DNA segment comprising a nucleotide sequence coding for the fused protein, producing and accumulating the fused protein in a culture, collecting the fused protein, and subjecting the collected fused protein to purification comprising cationic exchange chromatography and gel permeation chromatography.

17. A method for preparing a recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising inserting the nucleotide sequence into a vector.

18. A method for preparing a transformant harboring a recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising transforming a microorganism with the recombinant DNA segment.

## Claims (Claims for the following Contracting State(s): ES)

1. A method for producing a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising cultivating a transformant harboring a recombinant DNA segment comprising a nucleotide sequence coding for the fused protein, producing and accumulating the fused protein in a culture, and collecting the fused protein.

2. A method for producing a fused protein in accordance with claim 1, wherein the heat-labile enterotoxin B subunit is fused with the herpes simplex virus surface antigen through a linker.

3. A method for producing a fused protein in accordance with claim 1, wherein the fused protein comprises herpes simplex virus surface antigen and heat-labile enterotoxin B subunit.

4. A method for producing a fused protein in accordance with claim 1, wherein the heat-labile enterotoxin B subunit is a product of enterotoxigenic Escherichia coli separated from a human.

5. A method for producing a fused protein in accordance with claim 1, wherein the herpes simplex virus surface antigen is gD or gB of herpes simplex virus type I or type II.

6. A method for producing a fused protein in accordance with claim 5, wherein the gD or gB is gD or gB lacking a transmembrane domain.

7. A method for producing a fused protein in accordance with claim 1, wherein the fused protein is a fused protein comprising gD lacking a transmembrane domain of herpes simplex virus type I and heat-labile enterotoxin B subunit.

8. A method for preparing a recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising inserting the nucleotide sequence into a vector.

9. A method for preparing a recombinant DNA segment in accordance with claim 8, wherein the fused protein is a fused protein comprising gD lacking a transmembrane domain of herpes simpilex virus type I and heat-labile enterotoxin B subunit.

10. A method for preparing a transformant harboring a recombinant DNA segment comprising a nucleotide sequence coding for a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising transforming a microorganism with the recombinant DNA segment.

11. A method for preparing a transformant in accordance with claim 10, wherein the fused protein is a fused protein comprising gD lacking a transmembrane domain of herpes simplex virus type I and heat-labile enterotoxin B subunit.

12. A method for preparing a transformant in accordance with claim 10, wherein the prepared transformant has the characteristics of CHO-HDT-2-35 (FERM BP-2590).

13. A method for preparing a transformant in accordance with claim 10, wherein the prepared transformant has the characteristics of Sp-neo-HDT-13-71 (FERM BP-3071).

14. A method for purifying a fused protein comprising herpes simplex virus surface antigen and heat-labile enterotoxin B subunit or functional fragment thereof, wherein the herpes simplex virus surface antigen is arranged on the amino terminal side and the heat-labile enterotoxin B subunit is arranged on the carboxy terminal side, the method comprising cultivating a transformant harboring a recombinant DNA segment comprising a nucleotide sequence coding for the fused protein, producing and accumulating the fused proein in a culture, collecting the fused protein, and subjecting the collected fused protein to purification comprising cationic exchange chromatography and gel permeation chromatography.

## Patentansprüche (Patentansprüche für folgende(n) Vertragsstaat(en): AT, BE, DE, DK, FR, GB, GR, IT, NL, SE)

1. Fusionsprotein, umfassend ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist.

2. Fusionsprotein gemäß Anspruch 1, worin die hitzelabile Enterotoxin-B-Untereinheit über einen Linker mit dem Herpes Simplex Virus Oberflächenantigen fusioniert ist.

3. Fusionsprotein gemäß Anspruch 1, welches ein Fusionsprotein ist, das ein Herpes Simplex Virus Oberflächenantigen und eine hitzelabile Enterotoxin-B-Untereinheit umfaßt.

4. Fusionsprotein gemäß Anspruch 1, worin die hitzelabile Enterotoxin-B-Untereinheit ein Produkt von aus Menschen isolierten enterotoxinogenen Escherichia coli ist.

5. Fusionsprotein gemäß Anspruch 1, worin das Herpes Simplex Virus Oberflächenantigen gD oder gB von Herpes Simplex Virus Typ I oder Typ II ist.

6. Fusionsprotein gemäß Anspruch 5, worin das gD oder gB ein gD oder gB ist, dem eine Transmembrandomäne fehlt.

7. Fusionsprotein gemäß Anspruch 1, welches ein Fusionsprotein ist, das ein gD von Herpes Simplex Virus Typ I, dem die Transmembrandomäne fehlt, und eine hitzelabile Enterotoxin-B-Untereinheit umfaßt.

8. Rekombinantes DNA-Segment umfassend eine Nukleotid-Sequenz, die ein Fusionsprotein umfassend ein Herpes Simplex Virus Oberflächenantigen und eine hitzelabile Enterotoxin-B-Untereinheit oder ein funktionelles Fragment davon, worin das Herpes Simplex Virus Oberflächenantige am aminoterminalen Ende und die hitzelabile Enterotoxin-B-Untereinheit am carboxyterminalen Ende angeordnet ist, kodiert.

9. Rekombinantes DNA-Segment gemäß Anspruch 8, worin das Fusionsprotein ein Fusionsprotein ist, das ein gD von Herpes Simplex Virus Typ I, dem die Transmembrandomäne fehlt, und eine hitzelabile Enterotoxin-B-Untereinheit umfaßt

10. Transformant, der ein rekombinantes DNA-Segment trägt, welches eine Nukleotid-Sequenz umfaßt, die ein Fusionsprotein umfassend ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, kodiert.

11. Transformant gemäß Anspruch 10, worin das Fusionsprotein ein Fusionsprotein ist, das ein gD von Herpes Simplex Virus Typ I, dem die Transmembrandomäne fehlt, und eine hitzelabile Enterotoxin-B-Untereinheit umfaßt.

12. Transformant gemäß Anspruch 10, welcher die charakteristischen Eigenschaften von CHO-HDT-2-35 (FERM BP-2590) aufweist.

13. Transformant gemäß Anspruch 10, welcher die charakteristischen Eigenschaften von Sp-neo-HDT-13-71 (FERM BP-3071) aufweist.

14. Verfahren zur Herstellung eines Fusionsproteins umfassend ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, das Verfahren umfassend Kultivieren eines Transformanten, der ein rekombinantes DNA-Segment trägt, welches eine das Fusionsprotein kodierende Nukleotid-Sequenz umfaßt, Herstellen und Anhäufen des Fusionsproteins in der Kultur und Sammeln des Fusionsproteins.

15. Verfahren gemäß Anspruch 14, worin das Fusionsprotein ein Fusionsprotein ist, das ein gD von Herpes Simplex Virus Typ I, dem die Transmembrandomäne fehlt, und eine hitzelabile Enterotoxin-B-Untereinheit umfaßt.

16. Verfahren zum Reinigen eines Fusionsproteins umfassend ein Herpes Simplex Virus Oberflächenantigen und eine hitzelabile Enterotoxin-B-Untereinheit oder- ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, das Verfahren umfassend Kultivieren eines Transformanten, der ein rekombinantes DNA-Segment trägt, welches eine für das Fusionsprotein kodierende Nukleotid-Sequenz umfaßt, Herstellen und Anhäufen des Fusionsproteins in der Kultur, Sammeln des Fusionsproteins und Unterwerfen des gesammelten Fusionsproteins der Reinigung umfassend kationische Austauscher-Chromatographie und Gelpermeations-Chromatographie.

17. Verfahren zur Herstellung eines rekombinanten DNA-Segmentes umfassend eine Nukleotid-Sequenz, die ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, kodiert, das Verfahren umfassend das Insertieren der Nukleotid-Sequenz in einen Vektor.

18. Verfahren zur Herstellung eines Transformanten, der ein rekombinantes DNA-Segment trägt, welches eine Nukleotid-Sequenz umfaßt, die ein Fusionsprotein umfassend ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, kodiert, das Verfahren umfassend Transformieren eines Mikroorganismus mit dem rekombinanten DNA-Segment.

## Patentansprüche (Patentansprüche für folgende(n) Vertragsstaat(en): ES)

1. Verfahren zur Herstellung eines Fusionsproteins umfassend ein Herpes Simplex Virus Oberflächenantigen und eine hitzelabile Enterotoxin-B-Untereinheit oder ein funktionelles Fragment davon, worin das Herpes Simplex Viurs Oberflächenantigen am aminoterminalen Ende und die hitzelabile Enterotoxin-B-Untereinheit am carboxyterminalen Ende angeordnet ist, das Verfahren umfassend Kultivieren eines Transformanten, der ein rekombinantes DNA-Segment trägt, welches eine das Fusionsprotein kodierende Nukleotid-Sequenz umfaßt, Herstellen und Anhäufen des Fusionsproteins in der Kultur und Sammeln des Fusionsproteins.

2. Ein Verfahren zur Herstellung eines Fusionsproteins gemäß Anspruch 1, worin die hitzelabile Enterotoxin-B-Untereinheit über einen Linker mit dem das Herpes Simplex Virus Oberflächenantigen fusioniert ist.

3. Verfahren zur Herstellung eines Fusionsproteins gemäß Anspruch 1, worin das Fusionsprotein ein Herpes Simplex Virus Oberflächenantige und eine hitzelabile Enterotoxin-B-Untereinheit umfaßt.

4. Verfahren zur Herstellung eines Fusionsproteins gemäß Anspruch 1, worin die hitzelabile Enterotoxin-B-Untereinheit ein Produkt von aus Menschen isolierten enterotoxinogenen Escherichia coli ist.

5. Verfahren zur Herstellung eines Fusionsproteins gemäß Anspruch 1, worin das Herpes Simplex Virus Oberflächenantigen gD oder gB von Herpes Simplex Virus Typ I oder Typ II ist.

6. Verfahren zur Herstellung eines Fusionsproteins gemäß Anspruch 5, worin das gD oder gB ein gD oder gB ist, dem eine Transmembrandomäne fehlt.

7. Verfahren zur Herstellung eines Fusionsproteins gemäß Anspruch 1, worin das Fusionsprotein ein Fusionsprotein ist, das ein gD von Herpes Simples Virus Typ I, dem die Transmembrandomäne fehlt, und eine hitzelabile Enterotoxin-B-Untereinheit umfaßt.

8. Verfahren zur Herstellung eines rekombinanten DNA-Segmentes umfassend eine Nukleotid-Sequenz, die ein Herpes Simplex Virus Oberflächenantigen und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, kodiert, das Verfahren umfassend das Insertieren der Nukleotid-Sequenz in einen Vektor.

9. Verfahren zur Herstellung eines rekombinanten DNA-Segments gemäß Anspruch 8, worin das Fusionsprotein ein Fusionsprotein ist, das ein gD von Herpes Simplex Virus Typ I, dem die Transmembrandomäne fehlt, und eine hitzelabile Enterotoxin-B-Untereinheit umfaßt.

10. Verfahren zur Herstellung eines Transformanten, der ein rekombinantes DNA-Segment trägt, welches eine Nukleotid-Sequenz umfaßt, die ein Fusionsprotein umfassend ein Herpes Simplex Virus Oberflächenantigen und eine hitzelabile Enterotoxin-B-Untereinheit oder ein funktionelles Fragment davon, worin das Herpes Simplex Virus Oberflächenantige am aminoterminalen Ende und die hitzelabile Enterotoxin-B-Untereinheit am carboxyterminalen Ende angeordnet ist, kodiert, das Verfahren umfassend Transformieren eines Mikroorganismus mit dem rekombinanten DNA-Segment.

11. Verfahren zur Herstellung eines Transformanten gemäß Anspruch 10, worin das Fusionsprotein ein Fusionsprotein ist, das ein gD von Herpes Simplex Virus Typ I, dem die Transmembrandomäne fehlt, und eine hitzelabile Enterotoxin-B-Untereinheit umfaßt.

12. Verfahren zur Herstellung eines Transformanten gemäß Anspruch 10, worin der hergestellte Transformant die charakteristischen Eigenschaften von CHO-HDT-2-25 (FERM BP-2590) aufweist.

13. Verfahren zur Herstellung eines Transformanten gemäß Anspruch 10, worin der hergestellte Transformant die charakteristischen Eigenschaften von Sp-neo-HDT-13-71 (FERM BP-3071) aufweist.

14. Verfahren zum Reinigen eines Fusionsproteins umfassend ein Herpes Simplex Virus Oberflächenantige und eine hitzelabile Enterotoxin-B-Untereinheit oder ein funktionelles Fragment davon, worin das Herpes Simplex Virus Oberflächenantigen am aminoterminalen Ende und die hitzelabile Enterotoxin-B-Untereinheit am carboxyterminalen Ende angeordnet ist, das Verfahren umfassend Kultivieren eines Transformanten, der ein rekombinantes DNA-Segment trägt, welches eine für das Fusionsprotein kodierende Nukleotid-Sequenz umfaßt, Herstellen und Anhäufen des Fusionsproteins in der Kultur, Sammeln des Fusionsproteins und Unterwerfen des gesammelten Fusionsproteins der Reinigung umfassend kationische Austauscher-Chromatographie und Gelpermiations-Chromatographie.

## Revendications (Revendications pour l'(les) Etat(s) contractant(s) suivant(s): AT, BE, DE, DK, FR, GB, GR, IT, NL, SE)

1. Protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal.

2. Protéine de fusion conforme à la revendication 1, dans laquelle la sous-unité B de l'entérotoxine thermolabile est fusionnée avec l'antigène de surface du virus de l'herpes simplex par l'intermédiaire d'un élément de liaison.

3. Protéine de fusion conforme à la revendication 1, qui est une protéine de fusion comprenant un antigène de surface du virus de l'herpes simplex et la sous-unité B de l'entérotoxine thermolabile.

4. Protéine de fusion conforme à la revendication 1, dans laquelle la sous-unité B de l'entérotoxine thermolabile est un produit d'Escherichia coli entérotoxinogènes isolées chez l'homme.

5. Protéine de fusion conforme à la revendication 1, dans laquelle l'antigène de surface du virus de l'herpes simplex est un gD ou gB du virus de l'herpes simplex de type I ou de type II.

6. Protéine de fusion conforme à la revendication 5, dans laquelle le gD ou gB est un gD ou gB dépourvu d'un domaine transmembranaire.

7. Protéine de fusion conforme à la revendication 1, qui est une protéine de fusion comprenant un gD du virus de l'herpes simplex de type I, dépourvu d'un domaine transmembranaire, et la sous-unité B de l'entérotoxine thermolabile.

8. Segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal.

9. Segment d'ADN recombinant conforme à la revendication 8, la protéine de fusion étant une protéine de fusion comprenant un gD du virus de l'herpes simplex de type I, dépourvu d'un domaine transmembranaire, et la sous-unité B de l'entérotoxine thermolabile.

10. Transformant abritant un segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal.

11. Transformant conforme à la revendication 10, la protéine de fusion étant une protéine de fusion comprenant un gD du virus de l'herpes simplex de type I, dépourvu d'un domaine transmembranaire, et la sous-unité B de l'entérotoxine thermolabile.

12. Transformant conforme à la revendication 10, qui présente les caractéristiques de CHO-HDT-2-35 (FERM BP-2590).

13. Transformant conforme à la revendication 10, qui présente les caractéristiques de Sp-neo-HDT-13-71 (FERM BP-3071).

14. Procédé de production d'une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la culture d'un transformant abritant un segment d'ADN recombinant comprenant une séquence de nucléotides codant la protéine de fusion, la production et l'accumulation de la protéine de fusion dans la culture, et la récupération de la protéine de fusion.

15. Procédé conforme à la revendication 14, dans lequel la protéine de fusion est une protéine de fusion comprenant un gD du virus de l'herpes simplex de type I, dépourvu d'un domaine transmembranaire, et la sous-unité B de l'entérotoxine thermolabile.

16. Procédé de purification d'une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la culture d'un transformant abritant un segment d'ADN recombinant comprenant une séquence de nucléotides codant la protéine de fusion, la production et l'accumulation de la protéine de fusion dans la culture, la récupération de la protéine de fusion et le fait de soumettre la protéine de fusion récupérée à une purification comprenant une opération de chromatographie par échange de cations ou de chromatographie par perméation de gel.

17. Procédé de préparation d'un segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant l'insertion de la séquence de nucléotides dans un vecteur.

18. Procédé de préparation d'un transformant abritant un segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la transformation d'un microorganisme à l'aide du segment d'ADN recombinant.

## Revendications (Revendications pour l'(les) Etat(s) contractant(s) suivant(s): ES)

1. Procédé de production d'une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la culture d'un transformant abritant un segment d'ADN recombinant comprenant une séquence de nucléotides codant la protéine de fusion, la production et l'accumulation de la protéine de fusion dans la culture, et la récupération de la protéine de fusion.

2. Procédé de production d'une protéine de fusion, conforme à la revendication 1, dans lequel la sous-unité B de l'entérotoxine thermolabile est fusionnée avec l'antigène de surface du virus de l'herpes simplex par l'intermédiaire d'un élément de liaison.

3. Procédé de production d'une protéine de fusion, conforme à la revendication 1, dans lequel la protéine de fusion comprend un antigène de surface du virus de l'herpes simplex et la sous-unité B de l'entérotoxine thermolabile.

4. Procédé de production d'une protéine de fusion, conforme à la revendication 1, dans lequel la sous-unité B de l'entérotoxine thermolabile est un produit d'Escherichia coli entérotoxinogènes isolées chez l'homme.

5. Procédé de production d'une protéine de fusion, conforme à la revendication 1, dans lequel l'antigène de surface du virus de l'herpes simplex est un gD ou gB du virus de l'herpes simplex de type I ou de type II.

6. Procédé de production d'une protéine de fusion, conforme à la revendication 5, dans lequel le gD ou gB est un gD ou gB dépourvu d'un domaine transmembranaire.

7. Procédé de production d'une protéine de fusion, conforme à la revendication 1, dans lequel la protéine de fusion comprend un gD du virus de l'herpes simplex de type I, dépourvu d'un domaine transmembranaire, et la sous-unité B de l'entérotoxine thermolabile.

8. Procédé de préparation d'un segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant l'insertion de la séquence de nucléotides dans un vecteur.

9. Procédé de préparation d'un segment d'ADN recombinant, conforme à la revendication 8, la protéine de fusion étant une protéine de fusion comprenant un gD du virus de l'herpes simplex de type I, dépourvu d'un domaine transmembranaire, et la sous-unité B de l'entérotoxine thermolabile.

10. Procédé de préparation d'un transformant abritant un segment d'ADN recombinant, comprenant une séquence de nucléotides codant une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la transformation d'un microorganisme à l'aide du segment d'ADN recombinant.

11. Procédé de préparation d'un transformant, conforme à la revendication 10, la protéine de fusion étant une protéine de fusion comprenant un gD du virus de l'herpes simplex de type I, dépourvu d'un domaine transmembranaire, et la sous-unité B de l'entérotoxine thermolabile.

12. Procédé de préparation d'un transformant, conforme à la revendication 10, le transformant préparé présentant les caractéristiques de CHO-HDT-2-35 (FERM BP-2590).

13. Procédé de préparation d'un transformant, conforme à la revendication 10, le transformant préparé présentant les caractéristiques de Sp-neo-HDT-13-71 (FERM BP-3071).

14. Procédé de purification d'une protéine de fusion qui comprend un antigène de surface du virus de l'herpes simplex et la sous-unité B de l'entérotoxine thermolabile ou un fragment fonctionnel de celle-ci, et dans laquelle l'antigène de surface du virus de l'herpes simplex est placé du côté amino-terminal et la sous-unité B de l'entérotoxine thermolabile est placée du côté carboxy-terminal, ledit procédé comprenant la culture d'un transformant abritant un segment d'ADN recombinant comprenant une séquence de nucléotides codant la protéine de fusion, la production et l'accumulation de la protéine de fusion dans la culture, la récupération de la protéine de fusion et le fait de soumettre la protéine de fusion récupérée à une purification comprenant une opération de chromatographie par échange de cations ou de chromatographie par perméation de gel.
